# EUROPÄISCHE PATENTSCHRIFT

(11) **EP 2 075 097 B1**
(45) Veröffentlichungstag und Bekanntmachung des Hinweises auf die Patenterteilung: **30.06.2021**
(21) Anmeldenummer: 08104154.3
(22) Anmeldetag: 29.05.2008
(51) Int. Cl.: A61B 6/04

(54) **Positioniervorrichtung und Verfahren zum Positionieren einer Last sowie medizinische Diagnostik- und/oder Therapieanlage**
Positioning device and method of positioning a load as well as medical diagnostic and/or therapy device
Dispositif de positionnement et procédé de positionnement d'une charge et installation médicale de diagnostic et/ou de traitement

(30) Priorität: 05.06.2007 DE 102007026114
(43) Veröffentlichungstag der Anmeldung: 01.07.2009
(73) Patentinhaber: Varian Medical Systems Particle Therapy GmbH & Co. KG, 53842 Troisdorf (DE)
(72) Erfinder: Fahrig, Wolfram, 91301 Forchheim-Kersbach (DE); Herrmann, Klaus, 90403 Nürnberg (DE); Löseken, Jochen Miguel, 95445 Bayreuth (DE)
(74) Vertreter: Kirchner, Veit

(56) Entgegenhaltungen:
- EP-A1- 1 749 550
- DE-A1- 10 312 025
- DE-A1-102004 044 234
- DE-C1- 4 340 960
- JP-A- 2005 198 920
- US-A1- 2006 142 657
- None

## Beschreibung

Die Erfindung betrifft eine Positioniervorrichtung sowie ein Verfahren zum Positionieren einer Last, insbesondere zur Patientenpositionierung im Rahmen einer Partikeltherapie. Weiterhin betrifft die Erfindung eine medizinische Diagnostik- und/oder Therapieanlage, indem eine derartige Positioniervorrichtung eingesetzt wird.

Bei der Partikeltherapie, insbesondere von Krebserkrankungen, wird in einem geeigneten Beschleuniger ein Partikelstrahl beispielsweise aus Protonen oder Schwerionen erzeugt. Der Partikelstrahl wird in einem Strahlungskanal geführt und tritt über ein Austrittsfenster des Strahlungskanals in einen Bestrahlungs- bzw. Behandlungsraum ein.

Der Erfolg einer Tumorbehandlung hängt von der Genauigkeit der Tumorpositionierung ab. Die Positionierung eines Patienten hängt dabei von mehreren Faktoren ab. Unter anderem wird die Genauigkeit der Positionierung durch die Lagerung des Patienten sowie durch die Steifigkeit des Patientenlagerungssystems beeinflusst. Bei der Positionierung des Patienten können durch das Gewicht des Patienten elastische Verformungen auftreten, die zu Ungenauigkeiten bei der Positionierung führen können; dies wird beispielhaft in den Dokumenten JP 2005 198920 A und DE 43 40 960 C1 gezeigt.

Eine Möglichkeit, damit auftretende Probleme zu vermeiden, ist es, steifere Konstruktionen zu verwenden, um elastische Verformungen unter der Last des Patienten weitgehend zu vermeiden. Dies geht jedoch mit höheren Kosten ein her.

Alternativ ist es möglich, elastische Verformungen zu kompensieren. Um dies zu gewährleisten, ist es zum Beispiel möglich, indem bekannte Lastdaten dazu verwendet werden, eine vorhersehbare Verformung zu kompensieren. Hierdurch kann das System jedoch nicht flexibel eingesetzt werden, da die Lastdaten von vornherein genau bekannt sein müssen. Dies ist im Rahmen der Partikeltherapie durch die Unterschiedlichkeit der Belastung von Patient zu Patient nicht gegeben.

Eine andere Möglichkeit ist es, Sensoren - wie beispielsweise Kraft-Momenten-Sensoren - zur Ermittlung der Belastung einzusetzen. Derartige Systeme sind jedoch vergleichsweise teuer.

Es ist daher die Aufgabe der Erfindung eine Positioniervorrichtung und ein Verfahren zum Positionieren einer Last anzugeben, mit der die Ermittlung der Belastung auf einfache, flexible und kostengünstige Weise möglich ist. Weiterhin ist es die Aufgabe der Erfindung eine medizinische Diagnostik- und/oder Therapieanlage mit einer derartigen Positioniervorrichtung anzugeben.

Demnach wird die Erfindung durch eine Positioniervorrichtung nach Anspruch 1, durch ein Verfahren nach Anspruch 8 sowie durch eine medizinische Diagnostik- und/oder Therapieanlage nach Anspruch 16 gelöst.

Die erfindungsgemäße Positioniervorrichtung zum Positionieren einer Last weist auf:
- einen Motor zum Bewegen der Positioniervorrichtung,
- eine Messvorrichtung, die dem Motor zugeordnet ist und mit der Messdaten ermittelbar sind, die die Motorstromaufnahme durch den Motor bei der Positionierung der Last kennzeichnen, und
- eine Auswertungsvorrichtung zur Auswertung der Messdaten, die von der Messvorrichtung ermittelt worden sind, sodass durch die Auswertung die Belastung der Positioniervorrichtung durch die Last ermittelbar ist.

Der Erfindung liegt die Idee zu Grunde, dass über die Motorstromaufnahme die Kraft und/oder das Drehmoment, welche auf den Motor wirken, ermittelt werden können. Aufgrund der bekannten Geometrie der Positioniervorrichtung kann hierüber die Belastung der Positioniervorrichtung durch die Last ohne zusätzliche Sensoren ermittelt werden. Die Lastdaten der Last, wie beispielsweise das Gewicht und/oder die Lage der Last auf der Positioniervorrichtung, müssen dabei nicht vorbekannt sein, sondern können mithilfe der Positioniervorrichtung wenigstens teilweise ermittelt werden.

Aus der ermittelten Belastung der Positioniervorrichtung kann beispielsweise eine insbesondere elastische Verformung durch die Belastung quantitativ vorhergesagt werden. Dies kann beispielsweise über einen auf Erfahrungswerten beruhenden und/oder über einen durch Berechnung ermittelten Zusammenhang geschehen. Damit lässt sich die Abweichung einer tatsächlichen Position der Last von einer gewünschten Sollposition ermitteln. Insbesondere bei einem statischen Positionieren der Last ergeben sich so Vorteile. Beispielsweise kann ein Warnsignal abgegeben werden, wenn die ermittelte Belastung oberhalb eines Schwellenwertes liegt, d.h. wenn die zu erwartende elastische Verformung einen Toleranzbereich überschreitet.

In einer vorteilhaften Ausführung weist die Positioniervorrichtung zusätzlich auf:
- zumindest einen weiteren Motor zum Bewegen der Positioniervorrichtung,
- eine weitere Messvorrichtung, die dem weiteren Motor zugeordnet ist und mit der weitere Messdaten ermittelbar sind, die eine Motorstromaufnahme durch den weiteren Motor bei der Positionierung der Last kennzeichnen.

In diesem Fall ist die Auswertungsvorrichtung derart ausgebildet, dass die weiteren Messdaten zusammen mit den Messdaten bei der Auswertung berücksichtigt werden. Dadurch, dass die Motorstromaufnahme weiterer Motoren gemessen wird, lässt sich die Belastung der Positioniervorrichtung genauer ermitteln. Beispielsweise kann eine Belastung, d.h. bestimmte Lastdaten, redundant ermittelt werden. Es kann aber auch die Belastung genauer ermittelt werden, indem nun Größen ermittelbar sind, zu deren Ermittlung die Messdaten zumindest zweier unterschiedlicher Motoren notwendig sind.

In einer Weiterbildung weist die Positioniervorrichtung zusätzlich eine Steuerungsvorrichtung zum Positionieren der Positioniervorrichtung auf. Die Steuervorrichtung ist derart ausgebildet, dass eine Verformung mit Hilfe der ermittelten Belastung ausgleichbar ist.

Hierdurch ist es möglich, insbesondere bei einer statischen Positionierung der Last an einer gewünschten Sollposition, die Sollposition automatisch zu erreichen, selbst wenn eine Verformung der Positioniervorrichtung durch die Last auftreten sollte. Nachdem die Belastung ermittelt ist, lässt sich hieraus die elastische Verformung der Positioniervorrichtung bestimmen. Dazu kann beispielsweise ein auf Erfahrung und/oder auf Berechnung beruhender Zusammenhang verwendet werden. Anschließend lässt sich ein Kompensationssignal ermitteln, mit dem die Steuervorrichtung die Position der Positioniervorrichtung entsprechend korrigiert, so dass die gewünschte Sollposition erreicht wird.

In einer Ausführungsform werden mit der Auswertungsvorrichtung das Gewicht der Last und/oder die Position des Schwerpunktes der Last, also in die Position der Last in Bezug auf die Positioniervorrichtung, ermittelt.

In einer Ausführungsform ist die Positioniervorrichtung als mehrachsiger Roboterarm mit mehreren Gelenken ausgebildet. Dies ermöglicht eine besonders flexible Positionierung einer Last. Dadurch, dass bei mindestens einem Motor, der zum Bewegen eines der Gelenke eingesetzt wird, die Motorstromaufnahme gemessen wird, kann nun die Belastung der als Roboterarm ausgebildeten Positioniervorrichtung ermittelt werden und gegebenenfalls eine Verformung der Positioniervorrichtung durch die Last ausgeglichen werden.

In vorteilhafter Weise wird bei der Auswertung der gemessenen Messdaten zumindest eine Gelenkstellung berücksichtigt. Dies ist immer dann notwendig, wenn der Roboterarm bei der Positionierung derart flexibel eingesetzt wird, dass bei einem der Gelenke deutlich unterschiedliche Gelenkstellungen auftreten können. In diesem Fall kann über diese Gelenkstellung die Geometrie des Roboterarms ermittelt werden, was insbesondere bei der Ermittlung des Schwerpunktes der Last bzw. des Gewichts der Last notwendig ist. Wenn jedoch der Roboterarm zur Positionierung der Last stets ähnliche und nur gering voneinander abweichende Gelenkstellungen aufweist, kann auf die Berücksichtigung zumindest einer Gelenkstellung bei der Ermittlung der Last verzichtet werden, da sich die Geometrie des Roboterarms nur unwesentlich ändert.

Insbesondere ist die Positioniervorrichtung als Patientenpositioniervorrichtung zum Positionieren eines Patienten in einer medizinischen Anlage, insbesondere in einer Partikeltherapieanlage, ausgebildet. Hierdurch wird eine Lösung für das Problem geboten, einen Patienten möglichst genau in einer medizinischen Anlage zu positionieren, wodurch die Sicherheit der Anlage erhöht wird.

Das erfindungsgemäße Verfahren zum Betreiben einer Positioniervorrichtung zum Positionieren einer Last, weist folgende Schritte auf:
- Positionieren der Last durch Bewegen der Positioniervorrichtung mithilfe des zumindest einen Motors,
- Ermitteln von Messdaten, die eine Motorstromaufnahme durch den zumindest einen Motor bei der Positionierung der Last charakterisieren,
- Auswerten der ermittelten Messdaten derart, dass eine Belastung der Positioniervorrichtung durch die Last ermittelt wird,
und gegebenenfalls zusätzlich:
- Steuern der Positioniervorrichtung unter Verwendung der ermittelten Belastung der Positioniervorrichtung derart, dass eine Verformung durch die Belastung ausgeglichen wird.

Das Verfahren kann beispielsweise softwaregestützt auf einer Rechnereinheit implementiert werden, die insbesondere mit der Positioniervorrichtung zur Steuerung derselben verbunden ist. Bei dem Ermitteln der Belastung können als Lastdaten beispielsweise das Gewicht der Last und/oder die Position des Schwerpunktes der Last bestimmt werden.

Die ermittelte Belastung kann zusätzlich einer Plausibilitätsüberprüfung unterzogen werden, wodurch sich die Sicherheit des Verfahrens erhöht.

Das Verfahren kann in einer bevorzugten Ausführungsform dafür ausgebildet sein, bei einer als mehrachsiger Roboterarm ausgebildeten Positioniervorrichtung mit mehreren Gelenken die Belastung zu ermitteln. Insbesondere kann dabei zumindest eine Gelenkstellung berücksichtigt werden.

Insbesondere, wenn die Positioniervorrichtung mehrere Motoren aufweist, und bei den Motoren jeweils Messdaten ermittelt werden, die deren Motorstromaufnahme bei der Positionierung der Last charakterisieren, kann das Ermitteln der Belastung der Positioniervorrichtung durch die Last redundant erfolgen. Auch hierdurch erhöht sich die Sicherheit des Verfahrens. Beispielsweise kann ein Fehlersignal ausgegeben werden, wenn bei der redundanten Ermittlung eine Abweichung festgestellt wird, die außerhalb eines Toleranzbereichs liegt. Wenn dieses Fehlersignal vorliegt, deutet dies auf eine Fehlfunktion der Positioniervorrichtung hin, so dass eine Bewegung der Positioniervorrichtung aus Sicherheitsgründen beispielsweise blockiert werden kann.

In der erfindungsgemäßen medizinischen Diagnostik- und/oder Therapieanlage ist eine Positioniervorrichtung für einen Patienten in einem Untersuchungsraum bzw. Behandlungsraum angeordnet, wobei die Positioniervorrichtung nach einem der Ansprüche 1 bis 6 ausgebildet ist und/oder nach einem Verfahren nach Anspruch 7 bis 11 betrieben werden kann. Die medizinisehe Diagnostik- und/oder Therapieanlage kann für die Strahlentherapie, insbesondere für die Partikeltherapie, ausgebildet sein. Gerade hier ist eine besonders genaue Positionierung eines Patienten von entscheidender Bedeutung.

Ausführungsformen der Erfindung mit vorteilhaften Weiterbildungen gemäß den Merkmalen der Unteransprüche werden anhand der folgenden Zeichnung näher erläutert, ohne jedoch darauf beschränkt zu sein. Es zeigen:
Figur 1 einen schematischen Überblick über eine Partikeltherapieanlage,
Figur 2 eine als Roboterarm ausgebildete Positioniervorrichtung für einen Patienten, und
Figur 3 eine schematische Darstellung eines Verfahrens für die Ermittlung der Belastung einer Patientenpositioniervorrichtung.

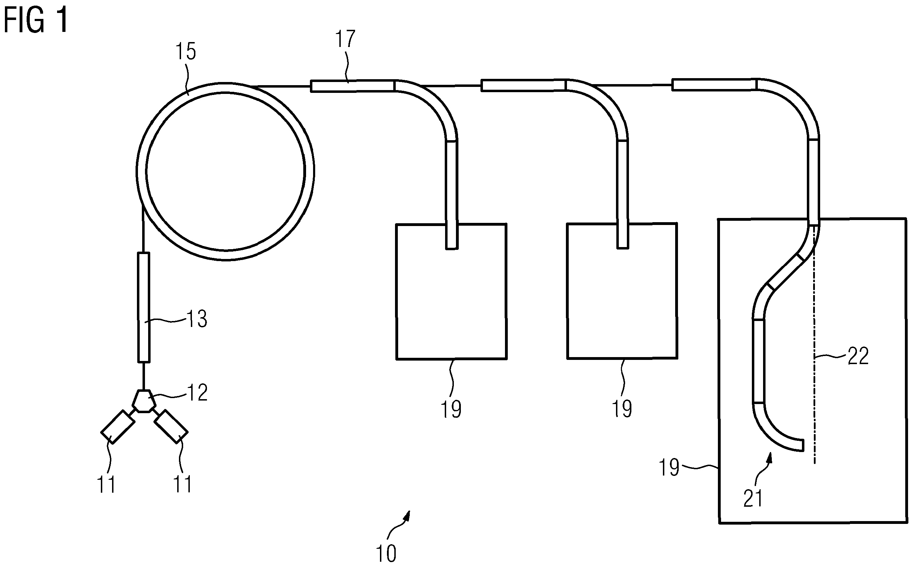

Figur 1 zeigt einen schematischen Überblick über den Aufbau einer Partikeltherapieanlage 10. In einer Partikeltherapieanlage 10 erfolgt eine Bestrahlung eines Körpers, insbesondere eines tumorerkrankten Gewebes, mit einem Partikelstrahl.

Als Partikel werden vornehmlich Ionen wie beispielsweise Protonen, Pionen, Heliumionen, Kohlenstoffionen oder andere Ionensorten eingesetzt. Üblicherweise werden derartige Partikel in einer Partikelquelle 11 erzeugt. Wenn, wie in Figur 1 dargestellt, zwei Partikelquellen 11 vorhanden sind, die zwei verschiedene Ionensorten erzeugen, kann zwischen diesen beiden Ionensorten innerhalb eines kurzen Zeitintervalls umgeschaltet werden. Dazu wird beispielsweise ein Schaltmagnet 12 verwendet, der zwischen den Ionenquellen 11 einerseits und einem Vorbeschleuniger 13 andererseits angeordnet ist. Z.B. kann hierdurch die Partikeltherapieanlage 10 mit Protonen und mit Kohlenstoffionen gleichzeitig betrieben werden.

Die von der oder einer der Ionenquellen 11 erzeugten und gegebenenfalls mit dem Schaltmagneten 12 ausgewählten Ionen werden in dem Vorbeschleuniger 13 auf ein erstes Energieniveau beschleunigt. Der Vorbeschleuniger 13 ist beispielsweise ein Linearbeschleuniger (LINAC für engl.: "LINear ACcelerator"). Anschließend werden die Partikel in einen Beschleuniger 15, beispielsweise ein Synchrotron oder Zyklotron, eingespeist. In dem Beschleuniger 15 werden sie auf hohe Energien, wie sie zur Bestrahlung nötig sind, beschleunigt. Nachdem die Partikel den Beschleuniger 15 verlassen, führt ein Hochenergiestrahl-Transportsystem 17 den Partikelstrahl zu einem oder mehreren Bestrahlungsräumen 19. In einem Bestrahlungsraum 19 werden die beschleunigten Partikel auf einen zu bestrahlenden Körper gerichtet. Je nach Ausgestaltung erfolgt dies von einer festen Richtung (in so genannten "fixed beam"-Räumen) aus oder aber über eine um eine Achse 22 bewegliche rotierbare Gantry 21 von verschiedenen Richtungen aus.

Der anhand der Figur 1 dargestellte Grundaufbau einer Partikeltherapieanlage 10 ist typisch für viele Partikeltherapieanlagen, kann aber auch hiervon abweichen. Die anhand der nachfolgenden Figuren beschriebene Positioniervorrichtung kann in einem der Bestrahlungsräume 19 zum Positionieren eines Patienten verwendet werden.

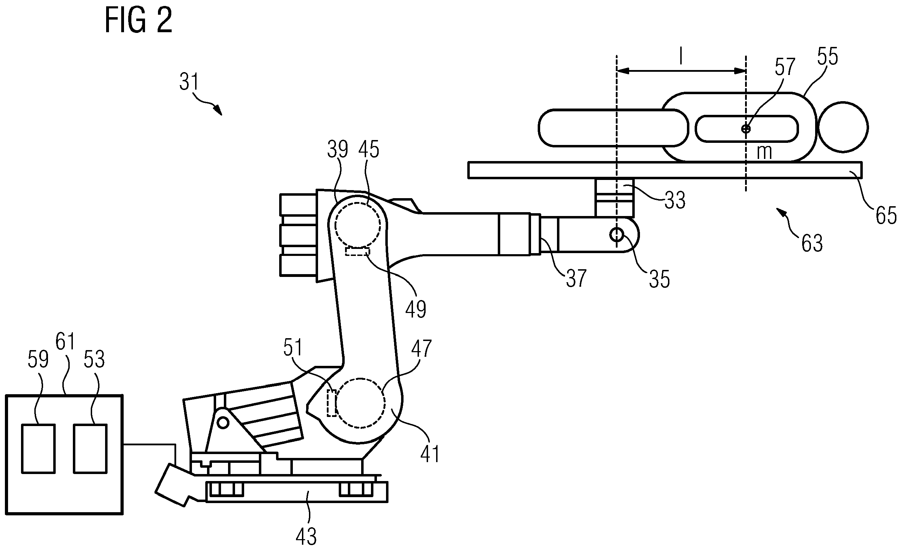

Figur 2 zeigt eine als Roboterarm 31 ausgebildete Positioniervorrichtung für einen Patienten 55, wie sie in einem Behandlungsraum einer Partikeltherapieanlage zum Einsatz kommt.

Der Roboterarm 31 weist dabei sechs verschiedene Gelenke 33, 35, 37, 39, 41, 43 auf, die jeweils von einem Motor 45, 47 bewegt werden. Die Motoren befinden sich dabei hinter den Verkleidungen des Roboterarms 31. An den Motoren 45, 47 sind jeweils Messvorrichtungen 49, 51 angeordnet, mit denen Messdaten ermittelt werden können, die die Motorstromaufnahme bei der Positionierung eines Patienten an dem jeweiligen Motor 45, 47 charakterisieren. Die Motoren 45, 47 sowie die zugeordneten Messvorrichtungen 49, 51 sind dabei lediglich an zwei Gelenken 39, 41 schematisch dargestellt, die übrigen Motoren und Messvorrichtungen sind der Übersichtlichkeit halber nicht wiedergegeben. Eine Messvorrichtung, die einem der Motoren zugeordnet ist, muss auch nicht wie dargestellt in unmittelbarer Nähe des Motors angeordnet werden. Sie kann beispielsweise auch in einer Steuerungseinheit für die Motoren angeordnet sein.

Die Messdaten werden an eine Auswertungsvorrichtung 53 weitergeleitet, mit der die Lastdaten der Positioniervorrichtung, also insbesondere das Gewicht m des Patienten 55 und die Lage l des Schwerpunktes 57 des Patienten 55, ermittelt werden können. Diese Lastdaten können in einem weiteren Schritt dazu verwendet werden, die Gelenke 33, 35, 37, 39, 41, 43 des Roboterarms 31 derart gegenzusteuern, dass Verformungen aufgrund einer Belastung der Positioniervorrichtung ausgeglichen werden können. Dies kann beispielsweise mit einer Steuervorrichtung 59 erfolgen, mit der eine Kompensationsbewegung des Roboterarms 31 passend durchgeführt werden kann.

Die Auswertungsvorrichtung 53 und die Steuervorrichtung 59 können dabei beispielsweise in einer mit dem Roboterarm verbundenen Rechnereinheit 61 realisiert sein.

Auf diese Weise kann beispielsweise eine Kompensation der Durchbiegung aller Komponenten des Tischsystems 63 durchgeführt werden, wie beispielsweise die Durchbiegung der Tischplatte 65, die Durchbiegung der Tischsäule, oder die Durchbiegung der Tischplatte inklusive angebautem Zubehör.

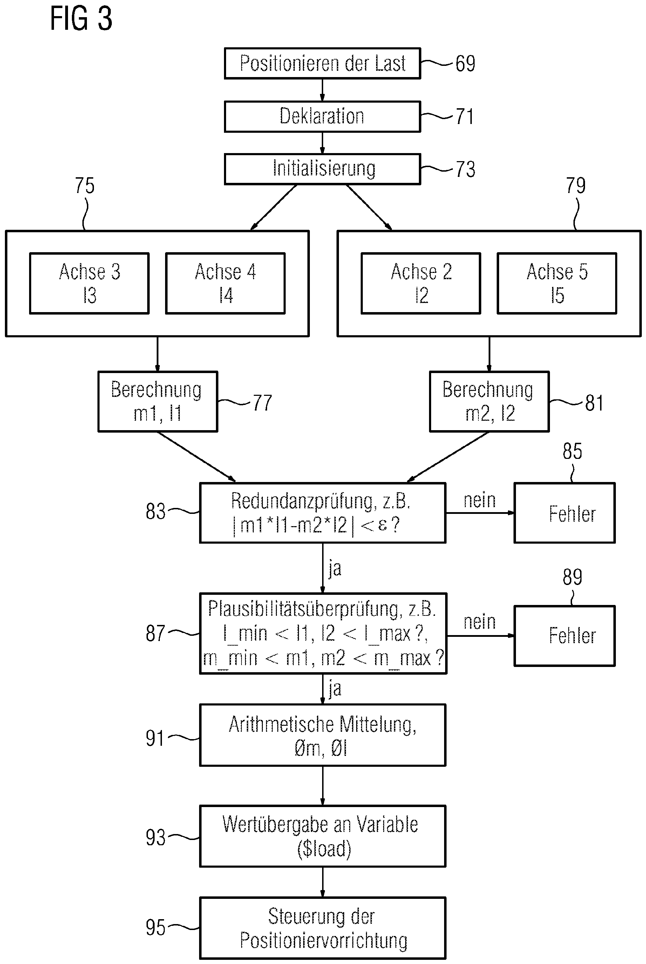

Figur 3 zeigt ein Flussdiagramm eine Ausführungsform des Verfahrens, bei dem die Belastung einer Positioniervorrichtung 31, wie sie in Figur 2 gezeigt ist, durch einen Patienten 55 ermittelt werden kann.

Dem Verfahren liegt die Idee zu Grunde, dass eine Proportionalität zwischen der Motorstromaufnahme Iₙ bei einem Gelenk n und dem auf das Gelenk wirkende Drehmoment Mₙ besteht. Das Drehmoment Mₙ ergibt sich wiederum aus der Geometrie des Roboters (Stellung der einzelnen Gelenke 33, 35, 37, 39, 41, 43) sowie den beiden Unbekannten l und m (Lage des Schwerpunktes des Patienten bzw. Gewicht des Patienten). Da die Geometrie, d.h. die Stellung der einzelnen Gelenke 33, 35, 37, 39, 41, 43, bekannt ist und die Motorstromaufnahme mit den Messvorrichtungen 49, 51 ermittelt werden kann, kann so die Belastung der Positioniervorrichtung durch einen Patienten während des Positionierens 69 ermittelt werden, wie im Folgenden näher ausgeführt wird.

Nach einer Deklaration 71 und einer Initialisierung 73 der für die Ermittlung der Belastung notwendigen Variablen erfolgt eine erste Messung 75 der Motorstromaufnahme an zwei verschiedenen Gelenken. Hieraus kann eine erste Berechnung 77 des Gewichts des Patienten m1 und der Lage des Schwerpunktes l1 des Patienten durchgeführt werden.

Parallel zu dieser ersten Messung 75 und ersten Berechnung 77 erfolgt eine zweite Messung 79 der Motorstromaufnahme an zwei weiteren, verschiedenen Gelenken. Analog zur ersten Berechnung 77 kann in einer zweiten Berechnung 81 das Gewicht des Patienten m2 und die Lage des Schwerpunktes l2 des Patienten errechnet werden.

Nach dieser zweifachen, redundanten Berechnung erfolgt eine Redundanzüberprüfung 83. Wenn sich die in der ersten Berechnung 77 und in der zweiten Berechnung 81 berechneten Größen zu sehr unterscheiden, beispielsweise wenn die Differenz der zugehörigen Drehmomente größer als ein vorgegebener Schwellenwert ε ist, deutet dies auf eine Fehlfunktion des Systems hin. In diesem Fall kann ein erstes Fehlersignal 85 ausgegeben werden.

Die berechneten Größen können gegebenenfalls einer Plausibilitätsüberprüfung 87 unterzogen werden, die immer dann ein zweites Fehlersignal 89 erzeugt, wenn einer der berechneten Werte außerhalb eines vorgegebenen Toleranzbereichs liegt, was ebenfalls auf eine Fehlfunktion des Systems deutet.

Wenn die Redundanzüberprüfung 83 bzw. die Plausibilitätsüberprüfung 87 keinen Fehler gemeldet hat, kann eine arithmetische Mittelung 91 des zweifach berechneten Gewichtes m1, m2 des Patienten bzw. des zweifach berechneten Schwerpunktes l1, l2 des Patienten durchgeführt werden.

Die gemittelten Größen werden als Variablen einer Rechnereinheit übergeben (Wertübergabe 93). Diese Variablen können beispielsweise dazu verwendet werden, eine Steuerung 95 will des Positioniersystems derart durchzuführen, dass eine Kompensation einer elastischen Verformung des Positioniersystems bei der Positionierung des Patienten durchgeführt wird.

Eine andere Möglichkeit ist beispielsweise eine Qualitätsüberprüfung des Positioniersystems im Rahmen einer beispielsweise täglich durchgeführten QA-Maßnahme. Hierbei wird das Positioniersystem mit einer definierten Last, d.h. mit vorbekannten Lastdaten, belastet. Das Verfahren wird durchgeführt und die an die Variablen übergebenen Werte können mit Referenzwerten verglichen werden. Immer dann, wenn sich die übergebenen Werte außerhalb eines vorgegebenen Toleranzbereiches befinden, deutet dies auf eine Fehlfunktion der Positioniervorrichtung hin.

## Patentansprüche

1. Positioniervorrichtung zum Positionieren einer Last in Form eines mehrachsigen Roboterarms mit mehreren Gelenken, aufweisend:
mindestens zwei Motoren (45, 47) zum Bewegen eines jeweiligen Gelenks des Roboterarms (31),
mindestens zwei Messvorrichtungen (49, 51), die jeweils einem zugehörigen der mindestens zwei Motoren (45, 47) zugeordnet und dazu ausgelegt sind, Messdaten zu ermitteln, die die Motorstromaufnahme durch den zugehörigen Motor bei der Positionierung der Last kennzeichnen,
eine Auswertungsvorrichtung (53) zur Auswertung der Messdaten, die von den mindestens zwei Messvorrichtungen (45, 47) ermittelt worden sind, um unter Berücksichtigung zumindest einer Gelenkstellung und der Annahme, dass eine Proportionalität zwischen der Motorstromaufnahme (In) bei einem Gelenk und dem auf das Gelenk wirkenden Drehmoment (Mn) besteht, die durch die Positionierung der Last (55) hervorgerufene Belastung der Positioniervorrichtung (31) zu ermitteln, wobei
die Auswertungsvorrichtung (53) dazu ausgelegt ist, die Position des Schwerpunktes (I) der Last während des Positionierens unter Berücksichtigung zumindest einer Gelenkstellung und der Annahme, dass eine Proportionalität zwischen der Motorstromaufnahme (In) bei einem Gelenk und dem auf das Gelenk wirkenden Drehmoment (Mn) besteht, zu ermitteln.

2. Positioniervorrichtung nach Anspruch 1,
wobei die Positioniervorrichtung (31) zusätzlich aufweist:
eine Steuerungsvorrichtung (59) zum Positionieren der Positioniervorrichtung derart, dass eine Verformung der Positioniervorrichtung durch die Last mit Hilfe der ermittelten Belastung ausgleichbar ist.

3. Positioniervorrichtung nach einem der vorhergehenden Ansprüche,
wobei die Auswertungsvorrichtung (53) dazu ausgelegt ist, das Gewicht (m) der Last zu ermitteln.

4. Positioniervorrichtung nach einem der vorhergehenden Ansprüche,
wobei die Positioniervorrichtung (31) als Patientenpositioniervorrichtung zum Positionieren eines Patienten (55) in einer medizinischen Anlage, insbesondere in einer Partikeltherapieanlage (10), ausgebildet ist.

5. Verfahren zum Betreiben einer Positioniervorrichtung zum Positionieren einer Last in Form eines mehrachsigen Roboterarms mit mehreren Gelenken, aufweisend folgende Schritte:
Positionieren der Last durch Bewegen des Roboterarms mithilfe zumindest zweier Motoren, von denen jeweils einer in einem jeweiligen Gelenk angeordnet ist,
Ermitteln von Messdaten, die eine jeweilige Motorstromaufnahme durch die zumindest zwei Motoren bei der Positionierung der Last charakterisieren,
Auswerten der ermittelten Messdaten derart, dass unter Berücksichtigung zumindest einer Gelenkstellung und der Annahme, dass eine Proportionalität zwischen der Motorstromaufnahme (In) bei einem Gelenk und dem auf das Gelenk wirkenden Drehmoment (Mn) besteht, eine durch die Positionierung der Last (55) hervorgerufene Belastung der Positioniervorrichtung und die Position des Schwerpunktes (I) der Last ermittelt wird.

6. Verfahren nach Anspruch 5, zusätzlich aufweisend folgenden Schritt:
Steuern der Positioniervorrichtung unter Verwendung der ermittelten Belastung der Positioniervorrichtung derart, dass eine Verformung der Positioniervorrichtung durch die Last ausgeglichen wird.

7. Verfahren nach Anspruch 5 oder 6, zusätzlich aufweisend folgenden Schritt:
Durchführen einer Plausibilitätsüberprüfung der ermittelten Belastung.

8. Verfahren nach einem der Ansprüche 6 bis 7,
wobei das Ermitteln der Belastung der Positioniervorrichtung das Ermitteln des Gewichts der Last und/oder der Position des Schwerpunktes der Last umfasst.

9. Verfahren nach einem der Ansprüche 5 bis 8,
wobei das Ermitteln der Belastung der Positioniervorrichtung durch die Last durch Auswerten der ermittelten Messdaten redundant erfolgt.

10. Verfahren nach Anspruch 9
wobei ein Fehlersignal ausgegeben wird, wenn bei der redundanten Ermittlung eine Abweichung festgestellt wird, die außerhalb eines Toleranzbereichs liegt.

11. Medizinische Diagnostik und/oder Therapieanlage mit einem Untersuchungsraum bzw. Behandlungsraum, indem eine Positioniervorrichtung für einen Patienten angeordnet ist, wobei die Positioniervorrichtung nach einem der Ansprüche 1 bis 4 ausgebildet ist.

12. Medizinische Diagnostik und/oder Therapieanlage nach Anspruch 11, wobei die medizinische Diagnostik und/oder Therapieanlage für die Strahlentherapie, insbesondere für die Partikeltherapie, ausgebildet ist.

## Claims

1. Positioning device for positioning a load in the form of a multiaxial robot arm with several articulations, comprising:
at least two motors (45, 47) for moving a respective articulation of the robot arm (31),
at least two measuring devices (49, 51), which are each assigned to an associated one of the at least two motors (45, 47) and are designed to determine measuring data that indicate the motor current consumption by the associated motor on positioning of the load,
an evaluation device (53) for evaluating the measuring data that have been determined by the at least two measuring devices (45, 47) in order to determine the loading of the positioning device (31) caused by the positioning of the load (55), taking account of at least one articulation position and the assumption that a proportionality exists between the motor current consumption (In) at an articulation and the torque (Mn) acting on the articulation, wherein
the evaluation device (53) is designed to determine the position of the centre of gravity (I) of the load during positioning, taking account of at least one articulation position and the assumption that a proportionality exists between the motor current consumption (In) at an articulation and the torque (Mn) acting on the articulation.

2. Positioning device according to claim 1,
wherein the positioning device (31) additionally comprises:
a control device (59) for positioning the positioning device in such a way that deformation of the positioning device by the load can be compensated for with the aid of the loading determined.

3. Positioning device according to one of the preceding claims,
wherein the evaluation device (53) is designed to determine the weight (m) of the load.

4. Positioning device according to any one of the preceding claims,
wherein the positioning device (31) is formed as a patient positioning device for positioning a patient (55) in a medical facility, in particular in a particle therapy facility (10).

5. Method for operating a positioning device for positioning a load in the form of a multiaxial robot arm with several articulations, comprising the following steps:
positioning the load by moving the robot arm by means of at least two motors, one of which is arranged in each articulation,
determining measuring data, which characterises respective motor current consumption by the at least two motors on positioning of the load,
evaluating the measuring data determined so that, taking account of at least one articulation position and the assumption that a proportionality exists between the motor current consumption (In) at an articulation and the torque (Mn) acting on the articulation, loading of the positioning device caused by the positioning of the load (55) and the position of the centre of gravity (I) of the load are determined.

6. Method according to claim 5, additionally comprising the following step:
controlling the positioning device using the determined loading of the positioning device such that deformation of the positioning device by the load is compensated for.

7. Method according to claim 5 or 6, additionally comprising the following step:
performing a plausibility check on the loading determined.

8. Method according to one of claims 6 to 7,
wherein determining the loading of the positioning device comprises determining the weight of the load and/or the position of the centre of gravity of the load.

9. Method according to any one of claims 5 to 8,
wherein determining the loading of the positioning device by the load takes place redundantly by evaluation of the measuring data determined.

10. Method according to claim 9,
wherein a fault signal is emitted if during the redundant determination a deviation is detected that lies outside a tolerance range.

11. Medical diagnostic and/or therapy facility with an examination room or treatment room in which a positioning device for a patient is arranged, wherein the positioning device is formed according to one of claims 1 to 4.

12. Medical diagnostic and/or therapy facility according to claim 11, wherein the medical diagnostic and/or therapy facility is formed for radiotherapy, in particular for particle therapy.

## Revendications

1. Dispositif de positionnement pour positionner une charge sous la forme d'un bras de robot multiaxial avec plusieurs articulations, présentant :
au moins deux moteurs (45, 47) pour déplacer une articulation respective du bras de robot (31),
au moins deux dispositifs de mesure (49, 51), qui sont associés respectivement à un moteur associé des au moins deux moteurs (45, 47) et sont conçus pour déterminer des données de mesure qui caractérisent la consommation de courant de moteur par le moteur associé lors du positionnement de la charge,
un dispositif d'évaluation (53) pour évaluer les données de mesure qui ont été déterminées par les au moins deux dispositifs de mesure (45, 47) afin de déterminer la contrainte, provoquée par le positionnement de la charge (55), exercée sur le dispositif de positionnement (31) en tenant compte d'au moins une position d'articulation et en supposant qu'il existe une proportionnalité entre la consommation de courant de moteur (In) pour une articulation et le couple de rotation (Mn) agissant sur l'articulation, dans lequel
le dispositif d'évaluation (53) est conçu pour déterminer la position du centre de gravité (I) de la charge pendant le positionnement en tenant compte d'au moins une position d'articulation et en supposant qu'il existe une proportionnalité entre la consommation de courant de moteur (In) pour une articulation et le couple de rotation (Mn) agissant sur l'articulation.

2. Dispositif de positionnement selon la revendication 1,
dans lequel le dispositif de positionnement (31) présente en supplément :
un dispositif de commande (59) pour positionner le dispositif de positionnement de telle manière qu'une déformation du dispositif de positionnement peut être compensée par la charge à l'aide de la contrainte déterminée.

3. Dispositif de positionnement selon l'une quelconque des revendications précédentes,
dans lequel le dispositif d'évaluation (53) est conçu pour déterminer le poids (m) de la charge.

4. Dispositif de positionnement selon l'une quelconque des revendications précédentes,
dans lequel le dispositif de positionnement (31) est réalisé en tant que dispositif de positionnement de patient pour positionner un patient (55) dans une installation médicale, en particulier dans une installation de traitement par particules (10).

5. Procédé pour faire fonctionner un dispositif de positionnement pour positionner une charge sous la forme d'un bras de robot multiaxial avec plusieurs articulations, présentant des étapes suivantes :
de positionnement de la charge en déplaçant le bras de robot à l'aide d'au moins deux moteurs, dont respectivement un est disposé dans une articulation respective,
de détermination de données de mesure qui caractérisent une consommation de courant de moteur respective par les au moins deux moteurs lors du positionnement de la charge,
d'évaluation des données de mesure déterminées de telle manière qu'une contrainte, provoquée par le positionnement de la charge (55), exercée sur le dispositif de positionnement et la position du centre de gravité (I) de la charge sont déterminées en tenant compte d'au moins une position d'articulation et en supposant qu'il existe une proportionnalité entre la consommation de courant de moteur (In) pour une articulation et le couple de rotation (Mn) agissant sur l'articulation.

6. Procédé selon la revendication 5, présentant en supplément l'étape suivante :
de commande du dispositif de positionnement en utilisant la contrainte déterminée exercée sur le dispositif de positionnement de telle manière qu'une déformation du dispositif de positionnement est compensée par la charge.

7. Procédé selon la revendication 5 ou 6, présentant en supplément l'étape suivante :
de mise en oeuvre d'une vérification de plausibilité de la contrainte déterminée.

8. Procédé selon l'une quelconque des revendications 6 à 7,
dans lequel la détermination de la contrainte exercée sur le dispositif de positionnement comprend la détermination du poids de la charge et/ou de la position du centre de gravité de la charge.

9. Procédé selon l'une quelconque des revendications 5 à 8,
dans lequel la détermination de la contrainte exercée sur le dispositif de positionnement par la charge est effectuée en évaluant les données de mesure déterminées.

10. Procédé selon la revendication 9,
dans lequel un signal d'erreur est envoyé quand est constaté, lors de la détermination redondante, un écart qui se situe en dehors d'une plage de tolérances.

11. Installation médicale de diagnostic et/ou de traitement avec un espace d'examen ou un espace de traitement dans lequel est disposé un dispositif de positionnement pour un patient, dans lequel le dispositif de positionnement est réalisé selon l'une quelconque des revendications 1 à 4.

12. Installation médicale de diagnostic et/ou de traitement selon la revendication 11, dans laquelle l'installation médicale de diagnostic et/ou de traitement est réalisée pour le traitement par rayons, en particulier pour le traitement par particules.
